(19) 

Europäisches Patentamt  
European Patent Office  
Office européen des brevets

(11) **EP 1 998 176 A1**

(12) **DEMANDE DE BREVET EUROPEEN**

(43) Date de publication:  
**03.12.2008 Bulletin 2008/49**

(51) Int Cl.:  
*G01N 33/28* (2006.01)  
*C02F 3/34* (2006.01)  
*C12M 1/34* (2006.01)  
*C12Q 1/64* (2006.01)

(21) Numéro de dépôt: **08290454.1**

(22) Date de dépôt: **14.05.2008**

(84) Etats contractants désignés:  
**AT BE BG CH CY CZ DE DK EE ES FI FR GB GR HR HU IE IS IT LI LT LU LV MC MT NL NO PL PT RO SE SI SK TR**  
Etats d'extension désignés:  
**AL BA MK RS**

(30) Priorité: **30.05.2007 FR 0703822**

(71) Demandeur: **IFP**  
**92852 Rueil-Malmaison Cédex (FR)**

(72) Inventeurs:  
• **Blanchet, Denis**  
**78110 Le Vésinet (FR)**  
• **Fleury, Marc**  
**78170 La Celle-Saint-Cloud (FR)**

(54) **Procédé de construction d'un milieu poreux adapté à l'étude de phénomènes de biodégradation**

(57) - Procédé de construction d'un milieu poreux adapté à l'étude de phénomènes de biodégradation.  
- On introduit dans une colonne de verre un mélange de sable et de terre, de façon progressive de manière à former un milieu poreux dont le réseau capillaire est homogène sur toute la hauteur de la colonne en terme de distribution de taille de pores. Puis on sature totalement ce milieu poreux par de l'eau, et l'on désature partiellement l'eau de façon homogène, en utilisant une membrane céramique microporeuse mouillable à l'eau et imperméable à l'air. Enfin, on sature les pores non remplis d'eau par de l'huile, puis l'on désature partiellement l'huile de façon homogène en utilisant une membrane céramique microporeuse mouillable à l'huile et imperméable à l'air.  
- Application notamment à l'étude de sols pollués ou de gisement d'hydrocarbures.

**Fig. 1**

EP 1 998 176 A1

**Description**

La présente invention concerne le domaine de l'évaluation de la biodégradation d'hydrocarbures piégés dans une structure géologique telle qu'un gisement pétrolier.

La méthode selon l'invention fournit un outil d'évaluation très utile notamment aux géologues soucieux d'orienter les investigations hors de zones à risque.

Un des problèmes couramment rencontrés lors de la définition de l'intérêt d'un objectif pétrolier, c'est-à-dire un piège à hydrocarbures non foré, situé à une relativement faible température (habituellement à moins de 80°C) est l'évaluation du risque de "biodégradation". En effet, il est couramment reconnu que la biodégradation, définie comme la destruction sélective d'une partie des molécules composant un brut pétrolier par des bactéries, peut se développer jusqu'à des températures pouvant atteindre 70 à 80°C. De telles températures sont courantes en domaine marin profond qui est une des zones où la recherche pétrolière est la plus active actuellement. Cette biodégradation, dont l'effet est généralement d'alourdir l'huile, d'augmenter sa viscosité et de diminuer son degré API, représente un risque majeur pour les compagnies pétrolières dont les forages en mer profonde représentent un investissement financier important. Toute méthode permettant de réduire ce risque est donc d'un intérêt majeur pour ces compagnies.

La biodégradation d'une huile, constituée de matière organique sous forme de molécules hydrocarbonées, est un phénomène d'altération provoquée par l'oxydation de certaines molécules hydrocarbonées par des micro-organismes ou flore bactérienne. Les bactéries consomment ces molécules hydrocarbonées dans le cadre de leur respiration et pour se procurer les éléments indispensables à leur croissance et leur réplication. La biodégradation conduit à la formation d'une huile lourde difficile à produire et commercialement moins rentable. L'étude de ce phénomène suscite un regain d'intérêt avec le développement de l'exploration dans les grands fonds où la présence d'huile lourde est un des risques majeurs. Il existe actuellement peu de moyens pour prédire les risques de biodégradation, alors que la nécessité économique de développement d'outils quantitatifs est de plus en plus forte.

**État de la technique**

Pour estimer les phénomènes de biodégradation aérobie d'une huile, il est connu de réaliser des mesures de respirométrie en conditions de laboratoire, où directement sur site par des mesures in situ. La respirométrie est une méthode d'analyse utilisée pour la détermination de l'activité métabolique par la mesure de la consommation d'oxygène. Ces mesures sont réalisées sur du sol maintenu en phase solide ou sur des colonnes reconstituant le milieu poreux et contenant de l'huile, de l'eau et de l'air. La mesure de l'activité de biodégradation se fait donc au niveau de l'échantillon entier, par le suivi de la consommation d'oxygène. Cette mesure d'oxygène permet un suivi cinétique beaucoup plus facilement que par le biais de l'analyse de la phase hydrocarbure, qui nécessite alors de multiplier les répliques de l'essai, qui sont sacrifiés pour chaque point de cinétique. Un impératif majeur pour l'utilisation de la respirométrie est de disposer d'échantillons de sols pollués sur lesquels les distributions respectives en hydrocarbures, en eau et en air seraient homogènes sur le profil vertical du milieu poreux, pour refléter une condition environnementale précise. C'est pourquoi on a très souvent recours, non pas à des échantillons de sol ou de sous-sols sous la forme de carottes, mais à des reconstitutions de sols pollués artificiellement par addition de quantités connues d'huile sur des sols.

Les études de biodégradation des hydrocarbures en milieu poreux reposent essentiellement sur des essais de laboratoire (dit en microcosmes) faciles à mettre en oeuvre. Ces essais, en batch ou en colonne, tentent de reproduire les conditions particulières de terrain à petite échelle. Les essais en batch sont des systèmes physiques dans lesquels un sol pollué pouvant associer le contaminant, les microorganismes (exogène ou indigènes), et le milieu minéral, sont réunis dans une fiole scellée. Le contenu en eau (milieu minéral) ne dépasse jamais le maximum de capacité de saturation (capacité au champs) pour éviter les conditions de milieu bi phasiques (saturation totale). Cependant les essais en batch présentent un inconvénient majeur qui est l'absence de structuration du milieu poreux, cette absence de structuration limitant notre connaissance de la distribution des différentes phases eau/huile/air au sein du milieu poreux. Cette connaissance est pourtant indispensable dans le cas de polluants ou de substrats carbonés non solubles, potentiellement biodégradables, pour lesquels les interfaces à l'eau (solubilisation et transfert inter-faciale) sont liées au réseau de capillarité développé au sein du milieu poreux étudié et aux valeurs de saturations partielles en eau et en huile.

Une alternative aux études en batch consiste à réaliser des études en colonne. De telles études sont décrites par exemple dans le document suivant : Wilkins, M.D., Abriola, L.M., Pennell, K.D. (1995). An expérimental investigation of rate-limited non-aqueous phase liquid volatilization in unsaturated porous media: steady state mass transfer. Water Resour. Res. 31(9), 2159-2172.

Le sol est alors mis en place en colonne, et de l'eau est additionné au système, soit en conditionnant du sol humide, soit en humidifiant partiellement le sol par deux étapes successives de saturation et de drainage. La difficulté majeure est la mise en place de la seconde phase hydrophobe, qu'est la phase hydrocarbure. L'hydrocarbure est souvent apporté en pied de colonne et transféré au sein du milieu poreux insaturé par volatilisation au travers de la phase gazeuse. De très rares essais proposent des milieux poreux insaturés avec une double insaturation (Wilkins et al., 1995).

Les processus de biodégradation aérobie d'une huile dans un milieu poreux se développent aux niveaux de l'interface eau/huile présente dans le milieu, le siège de la biodégradation se situant dans la phase eau. L'oxygène nécessaire est transféré de la phase gaz vers la phase eau (nécessité d'une interfaces air/eau). L'hydrocarbure est transféré de la phase huile vers la phase eau dans le cas de composés solubles dans l'eau, ou est dégradé au niveau de l'interface entre l'eau et l'huile pour les composés, non ou très peu, solubles (dégradation interfaciale).

L'activité biologique dans le milieu poreux est donc régie par - les caractéristiques des interfaces développées (liées à la granulométrie) et par - les phénomènes de diffusion (oxygène de la phase air vers la phase eau par exemple) et de solubilisation (transfert de composés hydrocarbures de la phase huile vers la phase eau). Elle s'opère en conséquence de manière identique en tout point de la colonne de milieu poreux, dès lors que cette dernière est homogène quant aux distributions respectives de l'huile, de l'eau et de l'air sur la hauteur de milieu poreux étudié.

La mesure de l'activité de biodégradation en respiromètrie se faisant par le suivi de la consommation d'oxygène au niveau global d'une colonne d'un milieu poreux, il est nécessaire de construire une colonne de milieu poreux dans laquelle les interfaces eau/huile/air sont maîtrisées de façon à être reproductibles et homogènes sur toute la hauteur de colonne, de façon à ce que le résultat de la biodégradation soit reproductible et représente une condition environnementale précise en tous points de la colonne de milieu poreux, en terme de granulométrie et de saturations partielles à l'eau et à l'huile.

L'objet de l'invention concerne un procédé permettant de constituer un milieu poreux adapté à l'étude des phénomènes cinétiques de biodégradation au sein d'un milieu souterrain. Le procédé repose sur la mise en place, pour une granulométrie donnée, d'une double saturation partielle à l'eau et à l'huile dans une colonne de matériau poreux, de façon à créer des interfaces eau/huile/air reproductibles et homogènes sur toute la hauteur de colonne.

Cette méthodologie rend possible l'utilisation de la technique de respirométrie pour mesurer la consommation d'oxygène au cours de la biodégradation sur la totalité du milieu poreux confectionné et permet de déduire des paramètres, comme des données cinétiques de dégradation d'une phase huile, pour une condition environnementale représentée par le milieu poreux constitué.

**Le procédé selon l'invention**

L'invention concerne un procédé de constitution d'un milieu poreux adapté à l'étude de phénomènes de biodégradation. Le procédé comporte les étapes suivantes :

- on introduit dans une colonne un mélange de matrice solide et d'inoculum bactérien de façon progressive de manière à former un milieu poreux homogène en terme de distribution de tailles de pores ;

- on sature totalement ledit milieu poreux par de l'eau, puis on désature partiellement l'eau en vidant l'eau d'au moins une partie des pores, à l'aide de moyens comportant une membrane microporeuse perméable à l'eau et imperméable à l'air de façon à ce que la désaturation soit homogène ;

- on remplit d'huile les pores ainsi vidés d'eau, puis on désature partiellement l'huile en retirant l'huile d'au moins une partie des pores remplis d'huile, à l'aide de moyens comportant une membrane microporeuse perméable à l'huile et imperméable à l'air de façon à ce que la désaturation soit homogène.

On peut utiliser des membranes microporeuses en céramique, que l'on positionne en aval d'un écoulement d'eau ou d'huile induit par le vidage d'eau, respectivement d'huile, lors de la désaturation partielle en eau, respectivement en huile.

Selon l'invention, on peut vider les pores en appliquant une dépression hydrostatique ou en appliquant une dépression au moyen d'une pompe à vide.

L'invention concerne également l'application du procédé à l'étude des phénomènes de biodégradation d'hydrocarbures dans un milieu souterrain. Au cours de cette application la granulométrie, le réseau poreux et les saturations en eau et en huile du milieu poreux sont choisis de façon à correspondre à ceux du milieu souterrain étudié. Cette application comporte la mesure de la consommation d'oxygène au cours de la biodégradation sur la globalité de la colonne de milieu poreux. Le milieu souterrain peut être un sol contenant des hydrocarbures, ou un réservoir d'hydrocarbures.

D'autres caractéristiques et avantages du procédé selon l'invention, apparaîtront à la lecture de la description ci-après d'exemples non limitatifs de réalisations, en se référant aux figures annexées et décrites ci-après.

**Présentation succincte des figures**

- la figure 1 montre le principe de désaturation qui s'effectue en installant une dépression par abaissement du réservoir par rapport au niveau de la céramique.

- la figure 2 illustre le principe de fonctionnement des céramiques, par la représentation schématique de l'évolution de la saturation en eau (Sw) de la céramique (C1) et du milieu poreux mis en place dans la colonne (C2) en fonction de la hauteur (H) de dépression imposée.

- la figure 3 illustre la mise en oeuvre des essais de biodégradation faite en connectant la colonne de milieu poreux (réacteur biologique) à une unité de mesure d'un respiromètre.

**Description détaillée du procédé**

Le procédé selon l'invention permet de constituer un milieu poreux adapté à l'étude des phénomènes cinétiques de biodégradation au sein d'un milieu souterrain.

**Principe**

Selon l'invention, on construit un modèle de sol constitué d'un milieu poreux ayant une granulométrie donnée, susceptible d'être le cadre de phénomènes de biodégradation. Pour ce faire, le milieu poreux construit comporte une matrice solide de granulométrie mono disperse et un inoculum bactérien (culture bactérienne sélectionnée de façon à déclencher un processus de conversion biologique). On appelle matrice solide un ensemble de grains de granulométrie unique et connue. Cette matrice peut être un sable (granulométrie de 80-120 $\mu$m par exemple) ou du carbure de silicium sable (granulométrie de 45/65 voire 10 $\mu$m par exemple) L'inoculum peut être de la terre. Dans ce cas la terre sert à apporter la microflore. Les proportions respectives du sable ou du carbure de silicium par rapport à la terre sont variables de 85/15 % à 95/5 % (en poids). Cette microflore peut également être introduite sous forme d'une suspension ou d'une culture en même temps que la phase eau, contenant les sels minéraux requis. Le milieu comporte ainsi une matrice solide (sable par exemple) et un inoculum bactérien (terre par exemple), auquel on ajoute de l'eau et des substrats hydrocarbonés (huile par exemple), et de l'air. Ainsi, au niveau des interfaces eau/huile se produit une activité de biodégradation que l'on peut mesurer.

Le milieu minéral salin utilisé pour la composition de la phase eau peut être de composition variable pour simuler les conditions environnementales. Il contient les éléments minéraux nécessaires à la croissance des bactéries apportées par l'inoculum. Les éléments minéraux essentiels sont l'azote et le phosphore. Le milieu aqueux est préférentiellement ajusté à pH 7 avant la mise en place de la phase aqueuse sur le massif sableux.

Les substrats hydrocarbonés susceptibles d'être utilisés sont peu limitatifs. Il peut s'agir de composés purs comme par exemple de l'hexadecane (un seul composé chimique). On peut également utiliser des mélanges de composés. On peut également utiliser des coupes industrielles représentant des coupes complexes associant des fractions saturées et des fractions aromatiques. Le 2, 2, 4, 4, 6, 8,8-heptaméthynonane (ou HMN) peut également être utilisé. Le HMN n'est pas dégradé par les bactéries. Il présente en conséquence l'avantage de simuler une phase huile dans laquelle on peut additionner à la fraction molaire désirée le ou les composés choisis pour l'étude. Il permet en conséquence de pouvoir étudier des phénomènes de biodégradation en maintenant une interface non réductible à zéro.

Ainsi, le procédé repose sur la mise en place, pour une granulométrie donnée, d'une double saturation partielle à l'eau et à l'huile dans une colonne de matériau poreux, de façon à créer des interfaces eau/huile/air reproductibles et homogènes sur toute la hauteur de colonne.

Mise en place de surfaces d'échange eau/huile/air reproductibles

Il est nécessaire de construire un milieu poreux dans lequel les interfaces eau/huile et eau /air sont respectivement reproductibles. Ces interfaces sont reproductibles si la surface d'échange que représentent ces interfaces est identique en forme et en superficie. Les processus de biodégradation dans les milieu poreux reposent en effet sur un certain nombre de paramètres physico-chimiques pressentis parmi lesquels les transferts d'oxygène de la phase air vers la phase eau, et de l'hydrocarbure de la phase huile vers la phase eau, qui dépendent de ces interfaces.

En maîtrisant les surfaces d'échanges, c'est-à-dire en reproduisant pour chaque expérience les mêmes surfaces d'échange, les conditions de transferts évoquées ci-avant sont identiques de ce point de vue. La mise en place d'un milieu poreux structuré non consolidé, partiellement saturé à l'eau et à l'huile, comporte deux étapes :

a) Construction d'un milieu poreux reproductible
Dans une première étape on construit un milieu poreux de façon à respecter une granulométrie et un réseau poreux fixés. Pour une granulométrie donnée, le but est d'être capable de reproduire un réseau poreux, c'est-à-dire de construire un milieu poreux ayant une distribution de tailles de pores comparable d'un essai à l'autre. La quantité de milieu poreux est définie, et permet d'offrir un volume poral (volume autour des grains de sol) et une taille moyenne de pores.
b) Mise en place de volumes d'eau et d'huile reproductibles
Dans cette seconde étape, ce volume de porosité bien défini est saturé partiellement en eau et en huile de façon à ce que l'eau occupe les pores les plus petits et l'huile les pores de tailles supérieures.

Ainsi, à l'issu de ces deux étapes, les deux phases eau et huile sont mises en place avec des volumes respectifs maîtrisés, dans un réseau poreux maîtrisé également (granulométrie et quantité). De ce fait, les interfaces eau/huile et eau/air sont reproductibles pour un milieu de granulométrie donnée et pour des saturations partielles bien définies d'eau et d'huile mis en place.

<u>Mise en place de surfaces d'échange eau/huile/air homogènes sur la hauteur de colonne</u>

Il est nécessaire de construire un milieu dans lequel les interfaces eau/huile/air sont homogènes en tout point sur toute la hauteur de milieu poreux. Ces interfaces sont homogènes si les saturations partielles respectives eau/huile/air du volume poral sont comparables en tous points du milieu poreux.

**Mise en oeuvre du procédé**

Ce procédé s'effectue séquentiellement selon les étapes suivantes :

    1- Installation d'un milieu poreux et étape de saturation à l'eau

    2- Étape de désaturation à l'eau

    3- Étapes de saturation et désaturation à l'huile

    4- Répartition de l'eau et de l'huile sur la hauteur du milieu poreux

La méthodologie est globalement la même pour les deux phases d'installation des phases eau et huile.

1- Installation d'un milieu poreux et étape de saturation à l'eau

Dans une première étape, on construit un milieu poreux de façon à respecter une granulométrie et un réseau poreux fixé. Pour une granulométrie données, le but est d'être capable de reproduire un réseau poreux, c'est-à-dire de construire un milieu poreux ayant une distribution de taille de pore identique. La distribution représente les fréquences d'apparition des différentes valeurs de tailles de pores présents dans le milieu.

Le milieu poreux est mis en place dans une colonne de verre comportant à sa base un collecteur (COL) en verre surmonté d'une céramique. Le maintien et l'étanchéité de la céramique et du collecteur sont assurés par serrage de cet ensemble sur une bague d'appui par la pression de serrage d'un bouchon percé. Le collecteur est relié par un tube flexible à un réservoir contenant le liquide de saturation, en l'occurrence l'eau ou un milieu salin défini.

Le remplissage du collecteur en verre est effectué en maintenant la colonne à un niveau plus bas que le réservoir d'eau et en desserrant le bouchon percé qui relie la base du collecteur au réservoir d'eau pour échapper l'air. La phase aqueuse est montée dans le collecteur de verre, de manière à être en affleurement de la céramique.

<u>Dans le cas d'un milieu poreux avec du sable de granulométrie 80-120 $\mu$m,</u> ou de carbure de silicium de granulométrie 40-60 $\mu$m, la mise en place du milieu poreux s'effectue à sec. Les quantités respectives de sable sont de 100 grammes, auquel on mélange jusqu'à 15 grammes de terre pour apporter l'inoculum bactérien. Dans le cas du carbure de silicium (CSi), les quantités de CSi sont de 140 grammes. Le milieu poreux ainsi préparé est mélangé manuellement dans un bécher au moyen d'une spatule. La mise en place du milieu poreux à sec est faite en introduisant progressivement le milieu poreux en pluie dans la colonne. La colonne de verre est tournée sur elle-même pendant le remplissage et la structuration du milieu est améliorée en frappant à la spatule les parois de la colonne de manière à obtenir un remplissage homogène. Cela permet de réaliser un milieu poreux d'une hauteur finie de l'ordre de 10 cm.

La saturation à l'eau du milieu poreux préalablement mis en place à sec se fait en établissant une différence de hauteur entre le bas de la colonne et le niveau d'eau du réservoir (charge ou dépression notée $\Delta H$) équivalent à 20 cm pour obtenir un débit modéré permettant une élimination optimale de la phase gazeuse présente dans le milieu poreux. La valeur de $\Delta H$ (dépression $-\Delta H$, charge $+\Delta H$) est par convention exprimée en précisant la différence de hauteur entre la solution aqueuse dans le réservoir et le niveau de la céramique dans la colonne. L'étape de saturation est arrêtée lorsque l'eau affleure à la surface du milieu poreux.

<u>Dans le cas de granulométrie plus fine,</u> la mise en place du milieu poreux s'effectue par voie humide en même temps que la phase de saturation à l'eau. L'apport du milieu poreux s'effectue sur une céramique mouillée sur la surface en contact avec le milieu poreux. On pratique un balayage à l'eau de la céramique en appliquant une faible charge $(-\Delta H)$ équivalente à 20 cm. Lorsque l'eau affleure à la surface de la céramique, une quantité de phase aqueuse de l'ordre de 5 ml est alors introduite dans la colonne au moyen d'une pipette. Le milieu poreux est ensuite versé en pluie dans la colonne de façon à remplir le volume d'eau. Cette séquence est renouvelée jusqu'à introduction de la totalité du milieu poreux. A chaque séquence de remplissage, on frappe avec une spatule les parois de la colonne de milieu poreux de manière à chasser les bulles d'air piégées dans le milieu au cours de cette étape de saturation à l'eau. Dans ce cas, la colonne est mise en attente pour éliminer l'excès d'eau au-dessus du milieu poreux.

A l'issue de l'étape de saturation à l'eau (étape 1) on accède à la détermination du volume poral développé par le milieu poreux.

2- Étape de désaturation à l'eau

A la fin de l'étape 1, le milieu est totalement saturé en eau. Il est donc nécessaire de réaliser une désaturation partielle en eau, de façon à obtenir, au final, un milieu partiellement saturé à l'eau.

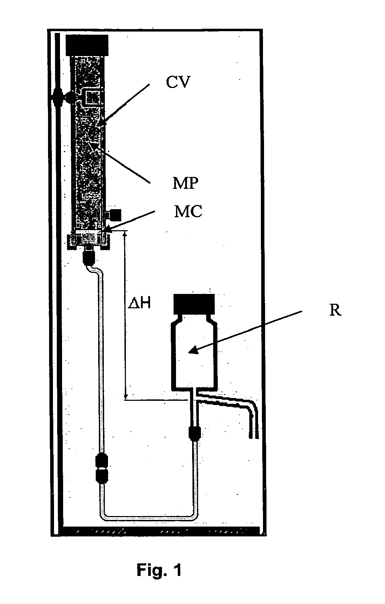

La désaturation peut s'effectuer en installant une dépression par abaissement du réservoir par rapport au niveau de la céramique, comme illustré sur la figure 1. Cette figure représente la colonne de verre (CV) contenant le milieu poreux (MP), et, à sa base, la céramique (MC). Plus généralement, on positionne la membrane microporeuse (MC) perméable à l'eau ou à l'huile, en aval de l'écoulement d'eau induit par le vidage d'eau lors de la désaturation partielle en eau ou en huile.

Un tube relie la céramique, par l'intermédiaire du collecteur à un réservoir (R). On peut pratiquer des paliers successifs de dépression pour atteindre la valeur finale désirée de désaturation.

La désaturation peut également s'effectuer par le biais d'une dépression effectuée par une pompe à vide. La dépression appliquée, mesurée par un manomètre, est réglée par une vis micrométrique. Une dépression de 1m d'eau est équivalente à une dépression de 100 mbar. Cette technique de désaturation par le biais d'une pompe à vide permet d'obtenir des valeurs de dépression importantes, nécessaires à la désaturation des milieux de granulométrie fine (10 μm par exemple). La valeur limite de dépression qui peut être obtenue par la pompe à vide est de 900 mbar. Dans tous les cas la désaturation à l'eau se traduit par une structuration du milieu poreux par collage des grains les uns aux autres.

On peut tracer des courbes de désaturation en eau du milieu poreux en fonction de la valeur de dépression appliquée. Ces courbes constituent de véritables abaques dépression/saturation partielle en eau pour une granulométrie de milieux poreux donnée. Ces abaques illustrent que les dépressions à appliquer pour désaturer partiellement un milieu poreux sont d'autant plus importantes que le milieu poreux est constitué par des faibles granulométries, les pores de grosse taille se vidant avec de plus faibles valeurs de dépression que les pores de plus petite taille. Ces courbes montrent également qu'il y a une limite à la désaturation du milieu poreux. Au-delà d'une certaine valeur de dépression, qui est fonction de la granulométrie, la sortie d'eau n'est plus possible. Cela s'explique par la rupture de la continuité du film d'eau au niveau du réseau capillaire au sein du milieu poreux.

A l'issue de la désaturation en eau du milieu poreux on détermine le volume de phase aqueuse résiduelle en place sur le milieu poreux.

3- Étapes de saturation et désaturation à l'huile

L'installation de la phase huile se fait sur un milieu poreux partiellement saturé en eau. La céramique mouillée à l'eau est retirée et remplacé par une céramique mouillée à l'huile. Cette opération est délicate et nécessite un maintien de la cohésion du milieu poreux au niveau de la zone de contact avec la céramique. La saturation à l'huile du milieu poreux est réalisée en appliquant une différence de hauteur (+ΔH de l'ordre de 20 cm). On laisse l'huile pénétrer progressivement par capillarité dans le milieu partiellement saturé en eau jusqu'à affleurement de la surface du milieu poreux. L'huile remplit les capillarités laissées libres par l'eau.

La désaturation s'effectue en installant une dépression par abaissement du réservoir par rapport au niveau de la céramique ou par le biais d'une pompe à vide, comme pour l'étape de désaturation à l'eau. On obtient le poids d'huile laissé en place dans le milieu poreux, et avec la densité, le volume d'huile mis en place.

On obtient une colonne de milieu poreux de granulométrie définie, avec un volume poral et un volume poreux (volume des grains plus volume poral) mesurés. On connaît les volumes d'eau et d'huile mis en place.

**Principe de la désaturation homogène**

Pour désaturer le milieu poreux, on applique une dépression à la base de la colonne (on pourrait appliquer une surpression d'air au sommet de la colonne, le but étant d'avoir une pression d'air homogène dans tout le volume poreux). L'air va ainsi traverser le milieu par un chemin préférentiel : l'air ne circule pas dans l'ensemble du milieu. Ainsi l'on ne pourra pas désaturer de façon homogène le milieu.

On souhaite donc appliquer une dépression uniforme sur tout le volume poreux. Selon l'invention, on résout ce problème en plaçant un matériau capable, dans certaines conditions, de bloquer l'air à la base de la colonne, mais de laisser passer l'eau ou, selon le cas, l'huile. De cette façon, l'air circule dans tout le volume poreux, désaturant ainsi de façon homogène le milieu poreux.

Ce matériau peut être une céramique dont les pores sont suffisamment petits pour laisser passer l'eau ou l'huile, mais pas l'air. Selon un exemple, la taille de pores de la céramique est telle que la pression de bulle eau-azote est de 1 bar, c'est-à-dire que l'air passe à travers la céramique à une pression de l'ordre de 1 bar, soit H=1m environ. Ainsi, lorsqu'une hauteur H inférieure à 1 m est appliquée, la céramique est toujours saturée à 100% et laisse donc passer uniquement l'eau. Par contre, la colonne poreuse se désature car la courbe de pression capillaire est plus faible. Pour une hauteur H donnée, on impose donc une saturation donnée jusqu'à un minimum appelé saturation irréductible. Si on tient compte de la hauteur de la colonne hc, il existe une faible différence de saturation entre le haut et le bas de la colonne, dépendant essentiellement de la répartition en tailles de grains.

Le caractère homogène de la répartition de l'eau et de l'huile en tout point du milieu poreux est donc obtenu par l'utilisation des céramiques microporeuses mouillables à l'eau et à l'huile.

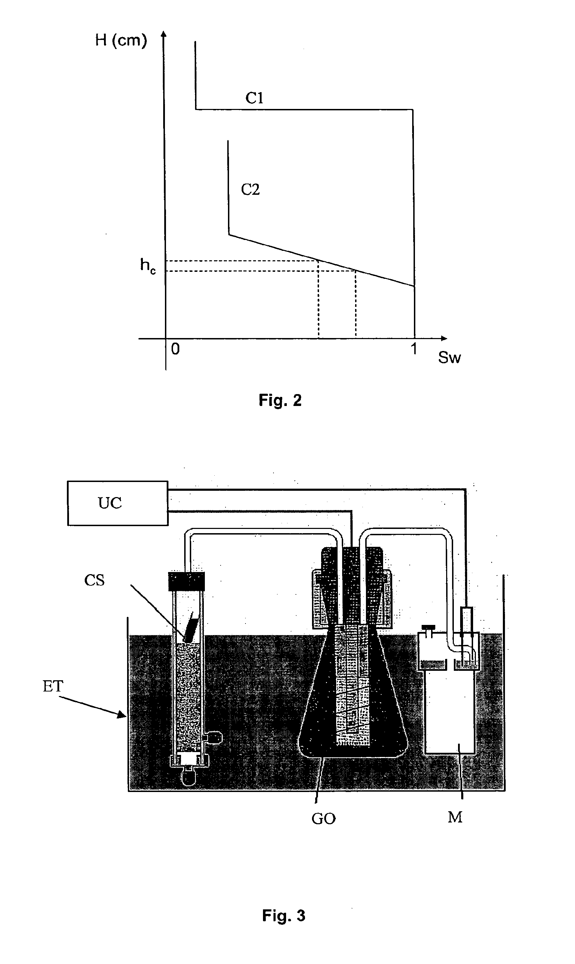

Ces céramiques sont primordiales pour établir des profils de saturation uniformes (ou quasi-uniformes) dans la colonne poreuse, et ainsi des surfaces d'échange homogènes le long de la colonne poreuse. Leur principe de fonctionnement peut être schématisé selon la figure 2 pour la mise en place de l'eau, la mise en place de l'huile étant similaire. Cette figure illustre l'évolution de la saturation en eau (Sw) en fonction de la hauteur H (induisant la dépression), pour la céramique (courbe C1) et pour la colonne poreuse (courbe C2). La céramique non traitée est un milieu poreux homogène mouillable à l'eau, dont le seuil de pression capillaire est élevé par rapport au milieu étudié. Dans l'expérience présente, la relation entre la pression capillaire et la hauteur H est :

$$Pc = \Delta\rho gH = \frac{2\sigma\cos\vartheta}{r}$$

où $\Delta\rho$ est la différence de densité entre l'air et l'eau, $\sigma$ la tension superficielle, $\vartheta$ l'angle de mouillage et $r$ la taille des seuils de pores.

Ces céramiques microporeuses utilisées sont identiques pour l'installation des phases eau et huile, mais les céramiques prévues pour l'installation d'une phase huile subissent un traitement hydrophobe pour les rendre mouillables à l'huile. Les céramiques destinées à l'installation de la phase eau ne sont pas traitées. Un exemple de céramiques microporeuses utilisées est décrit ci-dessous :

Tailles de pores de 2,6 $\mu$m (pression de bulle d'environ 1 bar)

Diamètre de la céramique de 35 +/ -0,5 mm

Épaisseur de céramique de 4 +/ -0,5 mm

Parallélisme de 0,02 m

Les céramiques sont ensuite saturées avec le fluide correspondant à leur mouillabilité : saturation à l'eau pour les céramiques hydrophiles et saturation à l'huile pour les céramiques hydrophobes par une technique de saturation sous vide.

Selon le procédé, on désature un milieu poreux, aussi bien à l'eau qu'à l'huile, en réglant les quantités d'eau et d'huile qui peuvent être retirées. Dans notre approche expérimentale, pour se permettre d'utiliser la respirométrie qui va mesurer la consommation d'oxygène au niveau de la totalité de la colonne de milieu poreux, il apparaît essentiel de s'assurer que la désaturation partielle imposée à la fois pour l'eau et pour l'huile permet de respecter la distribution homogène, pour chacune de ces phases, sur la hauteur de colonne, sans frange capillaire en bas de colonne. Pour des valeurs de désaturation trop faibles, on est sujet à la présence de franges capillaires c'est-à-dire à avoir une saturation totale du volume poral à l'eau du bas de la colonne. Cette saturation n'est pas souhaitée techniquement pour pouvoir introduire l'huile ensuite. Sinon nous aurions une mobilisation de l'eau par un front d'huile. Nous connaissons donc pour une granulométrie donnée les désaturations imposées par des vides ($\Delta$H) (ou dépressions) nécessaires pour avoir une distribution de l'eau et de l'huile sans présence de franges capillaires en bas de colonne.

Ainsi, au cours de la désaturation l'eau se vide, sur toute la hauteur de colonne (la répartition de la taille des pores est supposée homogène en tous points de la colonne de milieu) en commençant par les pores les plus gros qui présentent des forces gravitaires et qui vont dans le sens de la vidange, puis par les pores plus petits et ainsi de suite. Quand on désature un milieu poreux, on vide progressivement du plus gros au plus petit pore.

Le même principe est utilisé pour ajuster la saturation en huile. Ceci est possible car l'eau a une mobilité faible à faible saturation. Après la mise en place de l'huile et l'installation d'une céramique mouillable à l'huile ne laissant passer que ce fluide, on opère de la même façon qu'avec l'eau. Dans la figure 2, la saturation en eau Sw peut être remplacée par la saturation en huile So et on peut ajuster la quantité d'huile en utilisant le même principe.

Après la mise en oeuvre du procédé, on peut vérifier l'homogénéité de la répartition de l'eau et de l'huile sur la hauteur de colonne : en tous points de la colonne de milieu poreux l'occupation du milieu poreux, en pourcentage du volume poral, pour une phase donnée, que ce soit l'eau, l'huile ou l'air est identique. Par exemple, on peut avoir 25, 35, et 40 % pour l'occupation globale du volume poral à l'eau, l'huile et l'air (ramenée à la colonne de milieu poreux) pour un certain niveau de la colonne. Ces mêmes pourcentages se retrouveront quelque soit le niveau.

Ainsi, les distributions respectives de l'eau, de l'huile et de l'air sont homogènes (grâce aux céramiques) à tous niveaux de la colonne de milieu poreux. Donc, les contraintes en termes de limitations des échanges de l'oxygène de la phase gaz vers la phase eau et de l'hydrocarbure de la phase huile vers la phase eau, sont individuellement, identiques en tous points de la colonne de milieu poreux. La phase eau est le siège de la biodégradation et nécessite pour un métabolisme aérobie à la fois de l'oxygène et une source carbonée, en l'occurrence l'hydrocarbure.

Comme cette distribution de chacune des phases air, eau, huile est homogène en tous points, la colonne de milieu poreux représente un point unique de fonctionnement de conditions environnementales de milieu poreux insaturé. On peut donc relier les résultats cinétiques obtenus à ce point de conditions environnementale, et utiliser une mesure globale de respirométrie pour représenter ce point de fonctionnement.

**Mise en oeuvre des essais de biodégradation**

Après saturation partielle à l'eau et à l'huile, la céramique mouillable à l'huile et le collecteur sont retirés et remplacés par une pièce en Téflon® venant s'appuyer à la base du milieu poreux. Cette pièce en Téflon® contribue à empêcher les entrées d'air à la base du milieu poreux et permet d'éliminer la céramique mouillée à l'huile.

## Couplage de la colonne de milieu poreux à un réacteur biologique

La mise en oeuvre des essais de biodégradation est faite en connectant la colonne de milieu poreux (réacteur biologique) à une unité de mesure d'un respiromètre. La figure 3 illustre cette mise en oeuvre. L'unité permet à la fois un apport en oxygène par l'intermédiaire d'un générateur d'oxygène (GO) et un suivi de la consommation en oxygène dans la colonne de milieu poreux (MP) par l'intermédiaire d'une unité de commande (UC) reliée à un système d'acquisition. L'ensemble est placé dans une enceinte thermostatée (ET).

Le principe de fonctionnement du respiromètre est le suivant. La colonne de milieu poreux est mise en communication avec le générateur d'oxygène. L'ensemble représente le compartiment d'essai qui est séparé d'un compartiment de référence intégrant une interface d'eau en U (manomètre - M). L'équilibre de pression entre le compartiment gazeux de référence et le ciel de la colonne (compartiment d'essai) est réalisé au démarrage de chaque essai par mise à l'atmosphère des deux compartiments gazeux. L'activité des microorganismes au sein du milieu poreux engendre une consommation d'oxygène et une production de $CO_2$. Le $CO_2$ est piégé par de la chaux sodée (CS) placée dans un réceptacle positionné dans le ciel de la colonne de milieu poreux. Dans ces conditions de piégeage du $CO_2$ l'activité métabolique génère une dépression au sein du ciel de la colonne, créant un déséquilibre au niveau du manomètre (M). Un contact au niveau du manomètre entraîne une stimulation du générateur d'oxygène permettant une production calibrée d'oxygène par voie électrolytique. Lorsque l'équilibre de pression est rétabli, le contact manométrique cesse et le générateur n'est plus stimulé. Le suivi de la consommation d'oxygène est faite par le système d'acquisition qui comptabilise le nombre de stimulations en fonction du temps.

Pour étudier un sol contenant des hydrocarbures, ou un réservoir d'hydrocarbures, sujet à des phénomènes de biodégradation d'hydrocarbures, on constitue, selon le procédé de l'invention, un milieu poreux homogène en tout point de la colonne de milieu poreux, aussi bien en terme de granulométrie, mais aussi de distributions respectives de l'eau, de l'air et de l'huile. La granulométrie, le réseau poreux et les saturations en eau et en huile de ce milieu poreux ainsi constitué sont choisis de façon à correspondre à ceux du milieu souterrain étudié (sol ou réservoir). Le milieu poreux reconstitué est donc, selon les saturations respectives en eau/air/huile et les granulométries, représentatif d'états de saturations partielles en eau, en huile et en air qui peuvent se rencontrer dans le sol naturel ou des réservoirs d'hydrocarbures. En conséquenc, on peut ainsi étudier ce qui se passe en terme de paramètre limitant de la biodégradation dans un milieu souterrain caractérisé par ces données physiques, qui représentent un point de fonctionnement.

## Revendications

1. Procédé de constitution d'un milieu poreux adapté à l'étude de phénomènes de biodégradation, dans lequel on introduit dans une colonne un mélange de matrice solide et d'inoculum bactérien, **caractérisé en ce que** l'introduction est progressive de manière à former un milieu poreux homogène en terme de distribution de tailles de pores, et **en ce que** le procédé comporte les étapes suivantes :

   - on sature totalement ledit milieu poreux par de l'eau, puis on désature partiellement l'eau en vidant l'eau d'au moins une partie des pores, à l'aide de moyens comportant une membrane microporeuse perméable à l'eau et imperméable à l'air de façon à ce que la désaturation soit homogène ;
   - on remplit d'huile les pores ainsi vidés d'eau, puis on désature partiellement l'huile en retirant l'huile d'au moins une partie des pores remplis d'huile, à l'aide de moyens comportant une membrane microporeuse perméable à l'huile et imperméable à l'air de façon à ce que la désaturation soit homogène.

2. Procédé selon la revendication 1, dans lequel on utilise des membranes microporeuses en céramique.

3. Procédé selon l'une des revendications précédentes, dans lequel on positionne ladite membrane microporeuse perméable à l'eau en aval d'un écoulement d'eau induit par le vidage d'eau lors de la désaturation partielle en eau.

4. Procédé selon l'une des revendications précédentes, dans lequel on positionne ladite membrane microporeuse perméable à l'huile en aval d'un écoulement d'huile induit par le vidage d'huile lors de la désaturation partielle en huile.

5. Procédé selon l'une des revendications précédentes, dans lequel on vide les pores en appliquant une dépression hydrostatique.

6. Procédé selon l'une des revendications précédentes, dans lequel on vide les pores en appliquant une dépression au moyen d'une pompe à vide.

7. Application du procédé selon la revendication 1 à l'étude des phénomènes de biodégradation d'hydrocarbures dans un milieu souterrain, dans laquelle la granulométrie, le réseau poreux et les saturations en eau et en huile dudit milieu poreux sont choisis de façon à correspondre à ceux du milieu souterrain étudié, et dans laquelle on mesure la consommation d'oxygène au cours de la biodégradation sur la globalité de la colonne de milieu poreux.

**8.** Application du procédé selon la revendication 7, dans laquelle ledit milieu souterrain est un sol contenant des hydrocarbures.

**9.** Application du procédé selon la revendication 7, dans laquelle ledit milieu souterrain est un réservoir d'hydrocarbures.

**Fig. 1**

**Fig. 2**

**Fig. 3**

**Office européen
des brevets**

**RAPPORT DE RECHERCHE EUROPEENNE**

Numéro de la demande

EP 08 29 0454

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | EP 0 605 308 A (INST FRANCAIS DU PETROLE [FR]) 6 juillet 1994 (1994-07-06) * exemples 13,24 * | 1-6 | INV.<br>G01N33/28<br>C02F3/34<br>C12M1/34<br>C12Q1/64 |
| Y | WILKINS ET AL: "An experimental investigation of rate-limited nonaqueous phase liquid volatilization in unsaturated porous media: Steady state mass transfer" WATER RESOURCES RESEARCH, AMERICAN GEOPHYSICAL UNION, US, vol. 31, no. 9, septembre 1995 (1995-09), pages 2159-2172, XP009094423 ISSN: 0043-1397 * page 2162, colonne de gauche, alinéa 3 - page 2163, colonne de gauche, alinéa 1; figure 1 * | 1-6 | |
| Y | RIIS V ET AL: "A simple closed system for the microbial degradation of volatile substances" BIOTECHNOLOGY TECHNIQUES, vol. 9, no. 10, 1995, pages 705-708, XP002464988 ISSN: 0951-208X * page 705, alinéa 2 - page 707, alinéa 3; figure 1 * | 7-9 | |
| Y | FR 2 888 251 A (INST FRANCAIS DU PETROLE [FR]) 12 janvier 2007 (2007-01-12) * abrégé * | 7,8 | |
| Y | FR 2 830 646 A (INST FRANCAIS DU PETROLE [FR]) 11 avril 2003 (2003-04-11) * abrégé * | 7,9 | |

DOMAINES TECHNIQUES RECHERCHES (IPC)

G01N
C02F
C12M
C12Q

Le présent rapport a été établi pour toutes les revendications

| Lieu de la recherche | Date d'achèvement de la recherche | Examinateur |
|---|---|---|
| Munich | 15 septembre 2008 | Lazar, Zala |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**

EP 08 29 0454

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-09-2008

| Document brevet cité au rapport de recherche | | Date de publication | Membre(s) de la famille de brevet(s) | | Date de publication |
|---|---|---|---|---|---|
| EP 0605308 | A | 06-07-1994 | CA | 2112638 A1 | 01-07-1994 |
| | | | DE | 69318958 D1 | 09-07-1998 |
| | | | DE | 69318958 T2 | 01-10-1998 |
| | | | DK | 605308 T3 | 12-10-1998 |
| | | | ES | 2119876 T3 | 16-10-1998 |
| | | | FR | 2699928 A1 | 01-07-1994 |
| | | | NO | 934857 A | 01-07-1994 |
| FR 2888251 | A | 12-01-2007 | DE | 102006031200 A1 | 18-01-2007 |
| | | | US | 2007007203 A1 | 11-01-2007 |
| FR 2830646 | A | 11-04-2003 | CA | 2462079 A1 | 17-04-2003 |
| | | | EP | 1436412 A2 | 14-07-2004 |
| | | | ES | 2268116 T3 | 16-03-2007 |
| | | | WO | 03031644 A2 | 17-04-2003 |
| | | | JP | 2005504977 T | 17-02-2005 |
| | | | US | 2005015228 A1 | 20-01-2005 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **WILKINS, M.D. ; ABRIOLA, L.M. ; PENNELL, K.D.** An expérimental investigation of rate-limited non-aqueous phase liquid volatilization in unsaturated porous media: steady state mass transfer. *Water Resour. Res.,* 1995, vol. 31 (9), 2159-2172